# EUROPEAN PATENT SPECIFICATION

(11) **EP 2 701 790 B1**
(45) Date of publication and mention of the grant of the patent: **20.04.2016**
(21) Application number: 11822252.0
(22) Date of filing: 01.09.2011
(51) Int. Cl.: A61N 1/05, A61N 1/378, A61N 1/36

(54) **INTRAVASCULAR ELECTRODES AND ANCHORING DEVICES FOR TRANSVASCULAR STIMULATION**
INTRAVASKULÄRE ELEKTRODEN UND VERANKERUNGSVORRICHTUNGEN FÜR TRANSVASKULÄRE STIMULATION
ÉLECTRODES INTRAVASCULAIRES ET DISPOSITIFS D'ANCRAGE POUR LA STIMULATION TRANSVASCULAIRE

(30) Priority: 11.07.2011 US 201161508165 P
(43) Date of publication of application: 05.03.2014
(73) Proprietor: Interventional Autonomics Corporation, Durham NC 27703 (US)
(72) Inventor: GLENN, Richard, A., Santa Rosa, CA 95403 (US); HOLBROOK, Kevin, Santa Rosa, CA 95403 (US); SMITH, Jeffrey, A., Santa Rosa, CA 95403 (US); ORTH, Geoffrey, A., Santa Rosa, CA 95403 (US); WILLIAMS, Michael, S., Santa Rosa, CA 95403 (US)
(74) Representative: Millburn, Julie Elizabeth
(86) International application number: PCT/US2011/001535
(87) International publication number: WO 2012/030393

(56) References cited:
- US-A1- 2006 293 741
- US-A1- 2006 293 741
- US-A1- 2007 083 194
- US-A1- 2007 083 194
- US-A1- 2007 255 379
- US-A1- 2007 255 379
- US-A1- 2008 161 803
- US-A1- 2008 161 803
- US-A1- 2010 023 088
- US-A1- 2010 023 088
- US-A1- 2010 204 560
- US-A1- 2010 204 560

## Description

### TECHNICAL FIELD OF THE INVENTION

The present application generally relates to intravascular electrodes, anchors, and associated systems and methods used for delivering therapy to nervous system targets. The invention is defined in the appended claims.

### BACKGROUND

Applicants' prior Application Publication No. U.S. 2007/0255379 A1 discloses an intravascular neurostimulation device and associated methods for using the neurostimulation device to stimulate nervous system targets. In various ones of the disclosed embodiments, electrodes positioned within a blood vessel (e.g. a jugular vein, superior vena cava, or inferior vena cava) are used to transvascularly stimulate nervous system targets located outside the vasculature. Such stimulation can be used to lower heart rate and/or control blood pressure as a treatment for hyperiension or heart failure (HF).

Applicants' prior Application Publication No. U.S. 2010/0023088 A1 discloses a method for transvascularly stimulating the vagus nerve and other nervous system structures, such as those disposed within the carotid sheath. The disclosed method includes advancing an energy delivery element, which may be an electrode, into an internal jugular vein, retaining the energy delivery element in a portion of the internal jugular vein contained within a carotid sheath, and energizing the energy delivery element to transvenously direct energy to target contents of the carotid sheath external to the internal jugular vein. The energy may be directed to a carotid artery within the carotid sinus sheath, and/or to a carotid sinus nerve or nerve branch within the carotid sinus sheath, to nerve branches emanating from carotid artery baroreceptors, and/or to a vagus nerve or associated nerve branch within the carotid sinus sheath. In some of the disclosed embodiments, a bi-lateral system is employed, in which a second electrode or other second energy delivery element is introduced into a second internal jugular vein and retained in a portion of the second internal jugular vein contained within a second carotid sheath. The second energy delivery element is energized to direct energy to contents of the second carotid sheath external to the second internal jugular vein.

U.S. 2010/0023088 A1 describes an electrode and anchor structure in which an anchor with electrodes coupled thereto is configured to allow partial deployment for mapping purposes before the anchor is fully deployed at its final location. The distal end of the anchor is contained within a tubular end cap during mapping. Once the anchor electrode position is omptimized, the anchor is fully deployed by detaching the tubular end cap from the distal end of the anchor thereby allowing the distal end of the anchor to fully expand.

The right vagus nerve primarily innervates the sinoatrial node of the heart; stimulation of this nerve increases the duration of the cardiac cycle. The left vagus nerve primarily innervates the atrioventricular (AV) node of the heart; stimulation of this nerve slows AV conduction. The inventors named herein conducted anatomical studies on human cadavers to investigate the relative location of the right vagus nerve to veins that could provide sites for transvenous vagal stimulation to reduce heart rate and blood pressure. The findings strongly support the rationale for a transvenous approach to vagus nerve stimulation in the human. The right vagus nerve and its cardiac branches closely and reliably course directly alongside the largest veins in the neck and superior mediastinum, namely the right internal jugular vein, right brachiocephalic vein, superior vena cava, and azygotic arch.

### BRIEF DESCRIPTION OF THE DRAWINGS

Fig. 1A is a perspective view showing a first embodiment of an electrode array together with a temporary anchor for use during mapping.
Fig. 1B is a side elevation view showing the array and anchor of Fig. 1A schematically disposed within a blood vessel.
Fig. 2A is similar to Fig. 1A but shows the anchor and array separated from one another.
Figs. 2B - 2D show the array of Fig. 2A with alternate temporary anchor designs.
Fig. 3 is a perspective view showing the Fig. 1A electrode array and the pinning anchor arranged with the vessel-wall contacting surface of the substrate facing upwardly, and with the opposite surface in contact with the anchor.
Figs. 4A and 4B are perspective views of a second embodiment of an array and anchor.
Fig. 5 is an exploded view of the system of Fig. 4A.
Fig. 6 is a distal end view of the system of Fig. 4A, with the anchor exploded from the substrate.
Fig. 7 shows the anchor of Fig. 4A mounted to a delivery mandrel. The substrate and electrodes are not shown for purposes of clarity.
Fig. 8 is similar to Fig. 7 and further shows a portion of the anchor retained on the mandrel by the inner sheath.
Fig. 9 is similar to Fig. 8, but the anchor is compressed within the outer sheath.
Fig. 10 is similar to Fig. 9 but illustrates withdrawal of the outer sheath to release the anchor.
Fig. 11A is a side elevation view of an alternative electrode and anchor configuration.
Fig. 11B is similar to Fig. 11A and shows the anchor exploded from the substrate.
Fig. 12A is a perspective view of the electrode and anchor of Fig. 11A.
Fig. 12B is similar to Fig. 12A and shows the anchor exploded from the substrate.

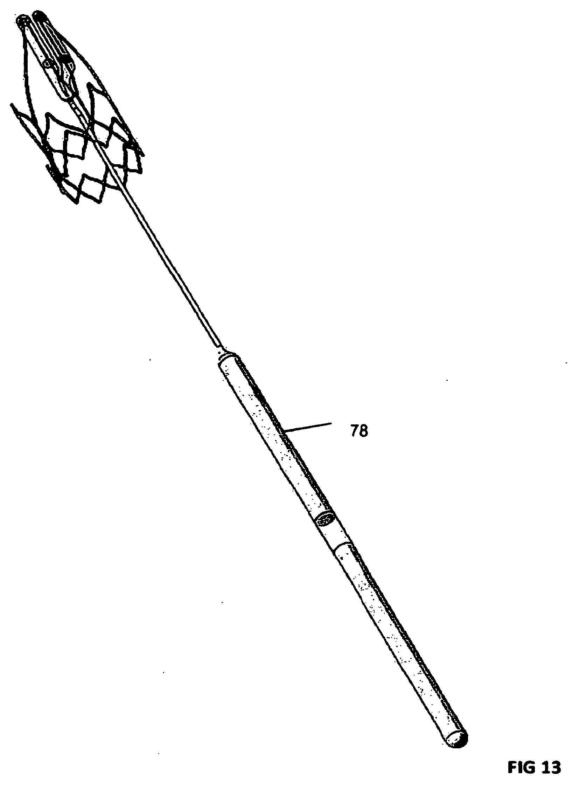

Fig. 13 illustrates the electrode and anchor of Fig. 11A coupled to an intravascular implant.
Fig. 14A illustrates the system of Fig. 11A with the first portion of the anchor deployed in the mapping position.
Fig. 14B is a perspective view of the anchor and electrodes of Fig. 11A with the second portion of the anchor deployed for chronic retention of the electrodes within the vasculature.
Fig. 14C is a perspective view of the distal ends of the tubular shaft and small diameter tube used for deploying the system of Fig. 11A.
Fig. 15 is a perspective view of a variation of the anchor and electrodes of Fig. 11A.
Fig. 16A is flattened view of a portion of a second variation of the anchor of Fig. 11B. Fig. 16B schematically shows the first portion of anchor of Fig. 16A deployed within a vessel to position electrodes in contact with the vessel wall for mapping.
Fig. 17A is flattened view of a portion of a third variation of the anchor of Fig. 11B. Fig. 17B schematically shows the first portion of anchor of Fig. 17A deployed within a vessel to position electrodes in contact with the vessel wall for mapping.
Fig. 18 shows an alternative substrate and electrode configuration that may be used for transvascular stimulation.
Fig. 19 schematically shows the Fig. 18 embodiment in a curled position within a vessel.
Fig. 20 is similar to Fig. 19 but does not show the vessel.
Figs. 21 are graphs illustrating data discussed herein.

### DETAILED DESCRIPTION

The present application describes designs of intravascular electrodes that may be positioned within the vasculature and used for stimulation of nervous targets. Also described are anchors suitable for temporarily holding the electrodes into contact with the vessel wall while mapping is performed to identify the optimal site for electrode placement, as well as anchors suitable for chronically retaining the electrodes at the optimal site once determined. The disclosed electrodes, anchors and associated components are suitable for use in fully intravascular systems of the type wherein both the electrodes and pulse generator are located within the vasculature, as well as in systems where the pulse generator is subcutaneously placed or located outside the body. The invention is set out in the appended claims. The embodiments, aspects or examples of the present description that do not fall within the scope of said claims are provided for illustrative purposes only and do not form part of the invention.

### First Embodiment

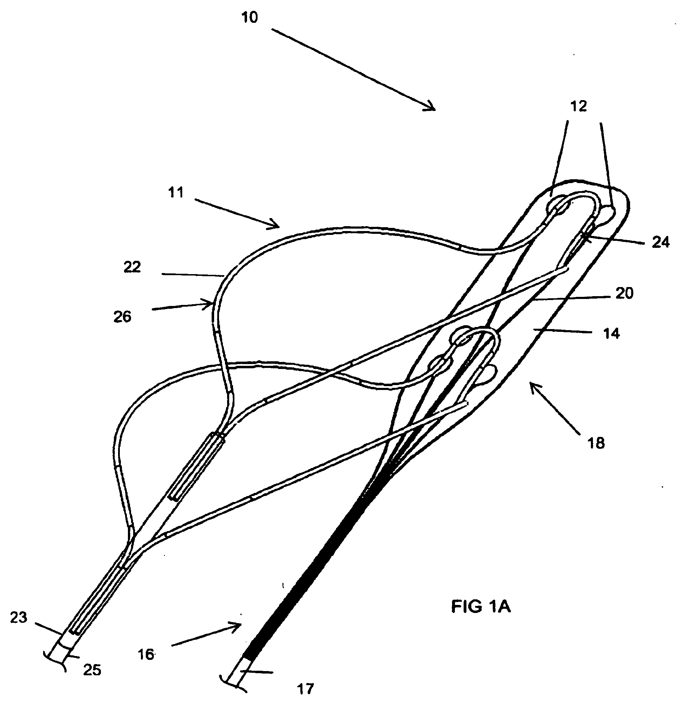

Fig. 1A illustrates a first embodiment of an electrode array 10 that is positionable within a blood vessel (such as the vessels discussed herein) and coupled to an intravascular, subcutaneous, or extracorporeal pulse generator (not shown) for use in transvascular stimulation. The array and associated pulse generator may be equipped to allow for programmable modulation of electrode selection in response to position-mediated signal adaptation. Array 10 comprises a plurality of electrodes 12 positioned on a flexible substrate 14. Four electrodes are shown in a 2x2 array, although various electrode numbers and arrangements may be used. The electrodes are positioned on the face of the substrate that faces the vessel wall when the system is implanted, thus placing the active electrode surfaces in contact with the vessel wall.

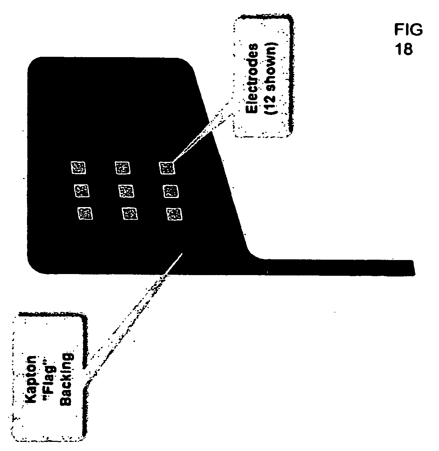

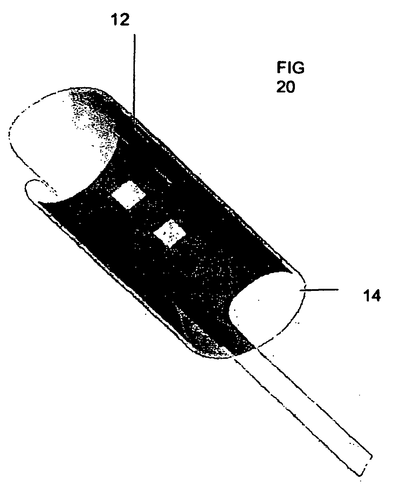

The substrate includes a relatively narrow portion 16, and a broader portion 18 (which may be paddle-like as shown) on which the electrodes are positioned. Although the longitudinal axes of the narrow and broader portions 18 are longitudinally aligned, in alternative embodiments the broader portion 18 may be positioned asymmetrically relative to the longitudinal axis of the narrow portion. Substrates and electrodes for alternative embodiments of this type are illustrated in Figs. 18 - 20.

In one embodiment, the electrodes are deposited on one face of the substrate 14, such that the substrate provides an electrically insulative backing and electrically isolates the electrodes from one another. In other embodiments, the electrodes may be positioned in or molded into the substrates or openings formed through the substrate. Conductors or conductive traces 20 are formed on or molded into the substrate and extend proximally from each of the electrodes 12, terminating at contacts near the proximal end of the narrow portion 16. A lead 17, which may be formed of tubing, shrink material, or other suitable material, is disposed over at least a portion of narrow portion 16, and includes conductors electrically coupled to the contacts of the conductors or traces 20.

The substrate is preferably a material capable of bending or curving relative to the vessel's longitudinal axis to approximately match or conform to the curvature of a blood vessel wall when held in contact with the wall by an anchor. The substrate will similarly be placed in a curved position when disposed within a delivery sheath for introduction into the vasculature. The substrate 14 may be a flex circuit formed of polyimide or other suitable materials. Alternate materials that may be used for the substrate include, but are not limited to, polyurethane, polyethylene, silicone rubber, fluoropolymer, stainless steel, platinum-iridium, MP35N, titanium and other biocompatible metals/polymers/elastomers. In some embodiments, all or a portion of the substrate may be of a type that resorbs or degrades over time, as tissue growth (e.g., cellular encapsulation, in-growth, endothelialization) begins to retain the electrodes in position. Materials suitable for this use include, but are not limited to, polylactide (PLA), polyglycolide (PGA) and their copolymer (PGLA). In such embodiments, the electrodes may be provided with non-degradable insulating material on the portions of the electrodes which are not intended for contact with the vessel wall, such that the insulating material remains intact following resorption or degradation of the substrate. In other embodiments, the flex circuit may be coated to improve its biocompatibility, to improve its insulative properties, and/or to reduce the body's response to a foreign substance. One preferred substrate is formed of polyethylene and has a covering or coating of silicone or polyurethane over the substrate and the conductors while leaving the electrodes themselves exposed.

For array implantation, it is desirable for the user to be able to empirically select an electrode location by positioning the electrode array, delivering stimulation from the selected location, measuring the response, and then repeating the process at one or more locations within the blood vessel. This mapping process allows the user to evaluate the response at various stimulation sites, so s/he may select the most optimal stimulation site for more permanent array positioning.

In the first embodiment, a temporary or mapping anchor 11 is positionable in contact with the vessel walls and the substrate 14 for use in retaining the array'.s position during mapping. After mapping, a second anchor is used to chronically retain the array such that the electrodes remain at the target site identified during mapping.

The anchor 11 biases the electrodes in contact with the vessel walls at the target site to as to ensure accurate mapping, but the radially outward forces imparted by the anchor against the surrounding vessel well are significantly lower than those imparted by the second anchor. The anchor 11 may be releasably attached to the substrate 14, chronically attached to the substrate using an adhesive or molded onto the substrate, or it may be separate from the substrate.

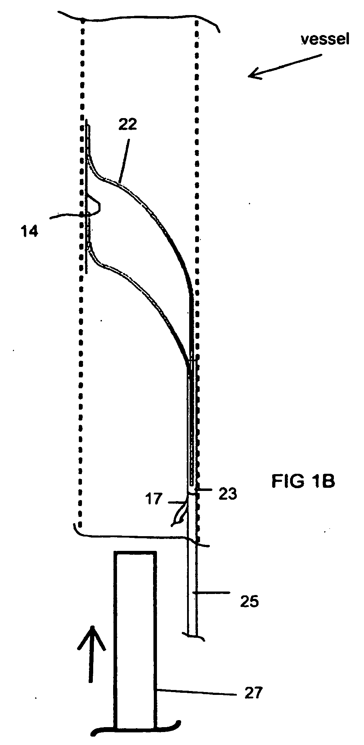

Referring to Fig. 1A, the anchor 11 may be formed of one or more loops 22 of resilient material including but not limited nitinol, stainless steel or resilient polymer. The loops 22 may be positioned on an elongate shaft 23. Each loop has a substrate contacting portion 24 and a vessel wall contacting portion 26 that extends away from the portion 24. The portion 26 is shaped to contact the vessel wall at one or more points so as to bias the electrodes 12 against the vessel wall as shown in Fig. 1B.

In one example shown in Figs. 1 - 2A, the substrate contacting portion 24 of a loop 22 may be a u-shaped tip portion defined by generally parallel wire sections occupying a plane. In this embodiment, the legs of the wire forming the loop extend proximally from the substrate and run adjacent (and preferably parallel) to one another near the point where they are connected to the shaft 23, allowing the legs to slightly cross-one another when the loop is deployed within a blood vessel. The ability of the legs to move slightly when the loop expands into contact with the vessel wall renders the loop 22 compatible with vessels having a range of different diameters.

A mandrel 25 is detachably coupled to shaft 23. The mandrel 25 has a proximal portion that, in use, extends outside the body so that the mandrel may be manipulated by the user to assist in positioning the temporary anchor and the substrate within the vasculature.

Although Fig. 1A shows narrow portion 16 of the substrate spaced from elongate shaft 23 and mandrel 25, the narrow portion 16 may alternatively be routed along an anchor loop 22 and elongate shaft 23 as shown in Fig. 1B.

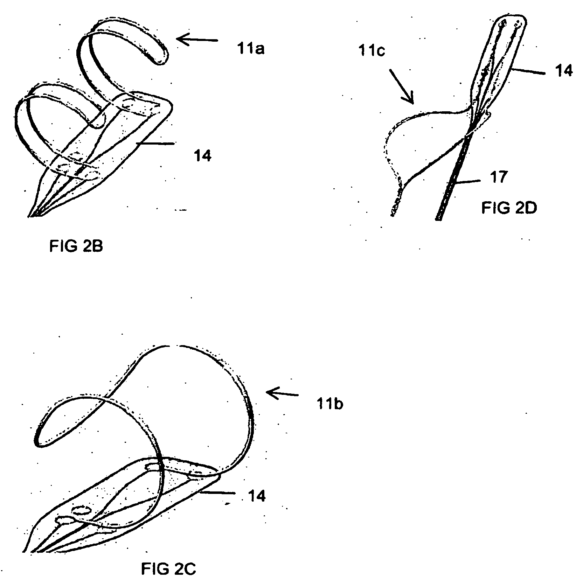

Other temporary anchor shapes include, but are not limited to, those shown in Figs. 2B - 2D. In the Fig. 2B embodiment, two anchors 11a are shown, each of which comprises a length of wire formed into a "U" shaped element. The wire ends forming the legs of the "U" are coupled to the substrate 14, spaced from one another (and from the legs of the other of the anchors) in a longitudinal direction relative to the substrate. The curved base of the "U" forms a free end of the anchor. The portion of each anchor extending from the legs to the base of the U curves around the longitudinal axis of the substrate so as to allow the anchor to circumferentially contact the vessel wall when deployed. Is this embodiment, the positioning mandrel (not shown) is detachably coupled to either or both of the anchors.

The Fig. 2C anchor 11b is similar to that of Fig. 2B but uses a single wire positioned with the legs of the "U" further apart along the longitudinal length of the substrate 14.

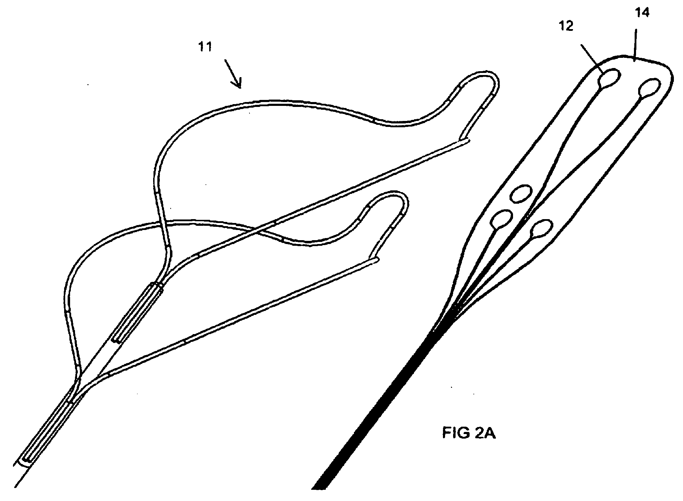

The Fig. 2D anchor 11c is similar to that of Fig. 2A, but uses a single anchor loop.

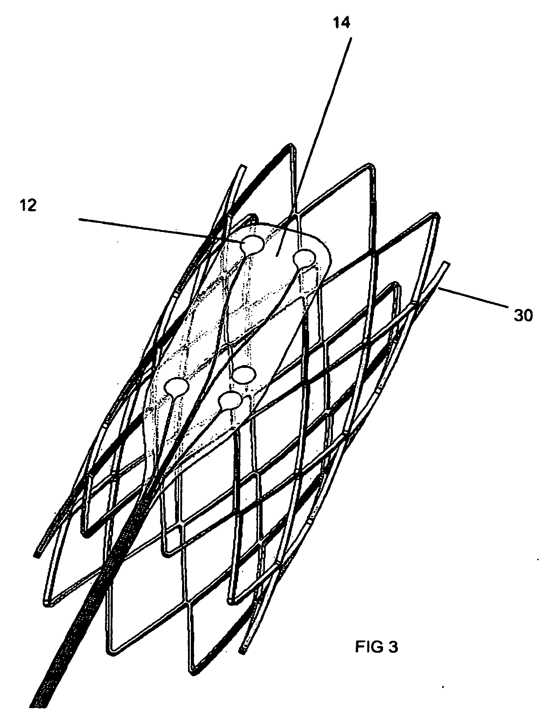

A second anchor 30 is provided for more permanently anchoring the array within the blood vessel once the optimal array position has been selected. As shown in Fig. 3, the second anchor 30 may be an expandable sleeve or stent-like device formed of nitinol, stainless steel, or other resilient material known for use in manufacturing stents. Second anchor 30 is configured to be advanced from a catheter 27 passed through the loops 22 of the temporary anchor as indicated by the arrow in Fig. 1B. The second anchor expands as it is advanced from the catheter 27, thus sandwiching the array between the anchor 30 and the vessel wall.

Prior to use, the positioning mandrel 25 is coupled to the temporary anchor and the array and temporary anchor are disposed within a delivery sheath (not shown). The sheath is percutaneously introduced into the vasculature using known methods, and then advanced to a desired location with a target vessel (e.g. the superior vena cava for vagus nerve stimulation). The array and temporary anchor are released from the sheath, such as by withdrawing the sheath while maintaining the position of the mandrel. The temporary anchor retains and biases the electrodes 12 in contact with the vessel wall. Mapping is performed by releasing and engaging the electrode against the vessel wall in this manner, then stimulating and observing the response. If additional mapping is deemed necessary, the procedure also includes recovering the array and anchor into the sheath by advancing the sheath distally while maintaining counter-traction on the mandrel. The sheath is advanced to another location and the process is repeated until the target location (at which the most optimal response to stimulation is measured) is identified.

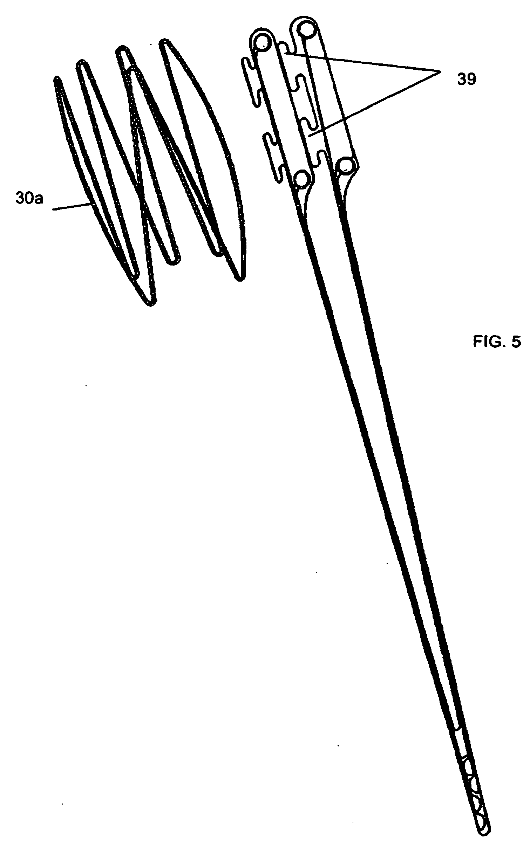

Once the target location is identified, the catheter 27 carrying second anchor 30 is passed through the loops 22 of the temporary anchor as indicated in Fig. 1B. The second anchor is released from the catheter 27 using methods known to those skilled in the art. The second anchor expands into contact with the inwardly-facing (relative to the vessel wall) of the substrate as shown in Fig. 5, and thus sandwiches the array between the anchor 30 and the vessel wall. The mandrel 25 is detached from the temporary anchor and is withdrawn from the body. The lead 17 may be attached to a pulse generator before or after placement of the electrodes. In one embodiment, the lead 17 is coupled to a fully intravascular pulse generator, such as is described in the aforementioned prior applications. Where the electrode array is to be deployed in the superior vena cava, the pulse generator may be deployed in the inferior vena cava, the superior vena cava, or elsewhere in the vasculature.

### Second Embodiment

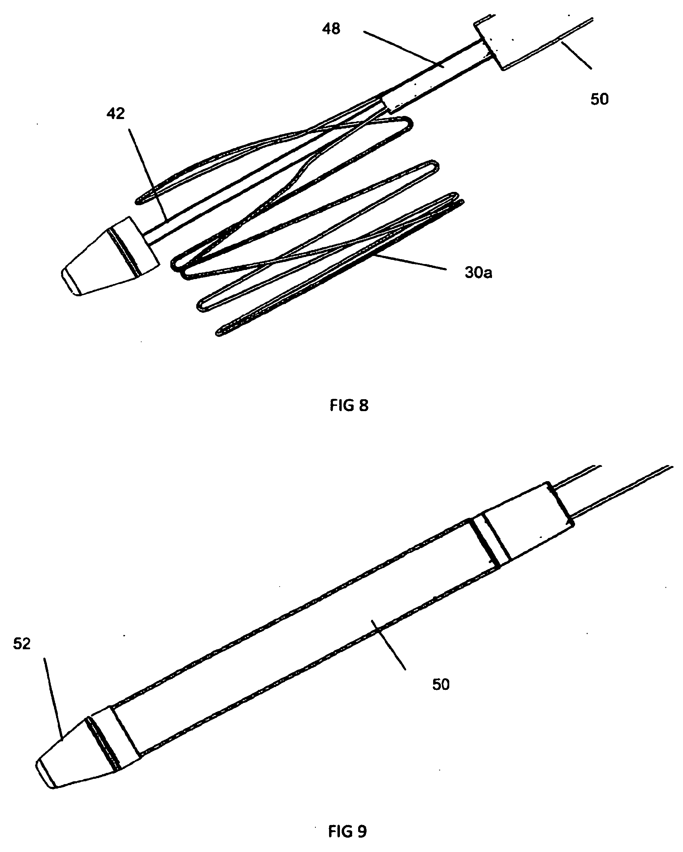

A second array system shown in Fig. 8, utilizes a self-expanding anchor 30a in combination with a flexible electrode array 32, which may be attached to the anchor or which may simply be positioned in contact with the anchor. In one form of this embodiment, the anchor 30a is attached to the array 32 and is employed for mapping as well as for chronically retaining the array within the vessel. The array includes two or more flexible, longitudinal, splines 34, each supporting a flexible circuit member or arm 36. Materials suitable for use in manufacturing the second embodiment include those listed for use with the first embodiment. For example, the members 36, and optionally the splines 34, may be formed of flexible materials of the type disclosed above in connection with the first embodiment of Fig. 1A. In one embodiment, the splines and the circuit arms are integral components of a single flexible substrate.

Two or more electrodes 38 are longitudinally arranged on each arm 36. As with the first embodiment, conductors or conductive traces are formed on or molded into the substrate and extend proximally from each of the electrodes, terminating at contacts near the proximal end of the narrow portion splines. A lead (not shown) which may be formed of tubing, shrink material, or other suitable material, is disposed over at least a proximal portion of the array (such as the portion of the substrate where the splines meet at the proximal end), and includes conductors electrically coupled to the contacts of the conductors or traces.

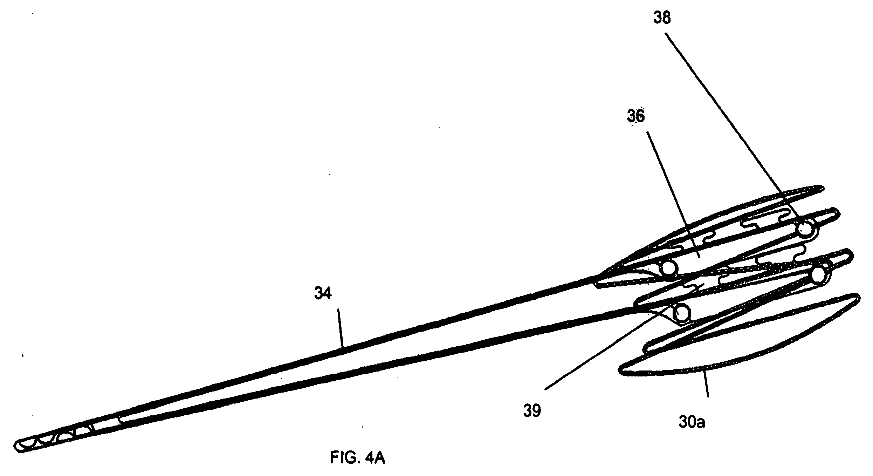

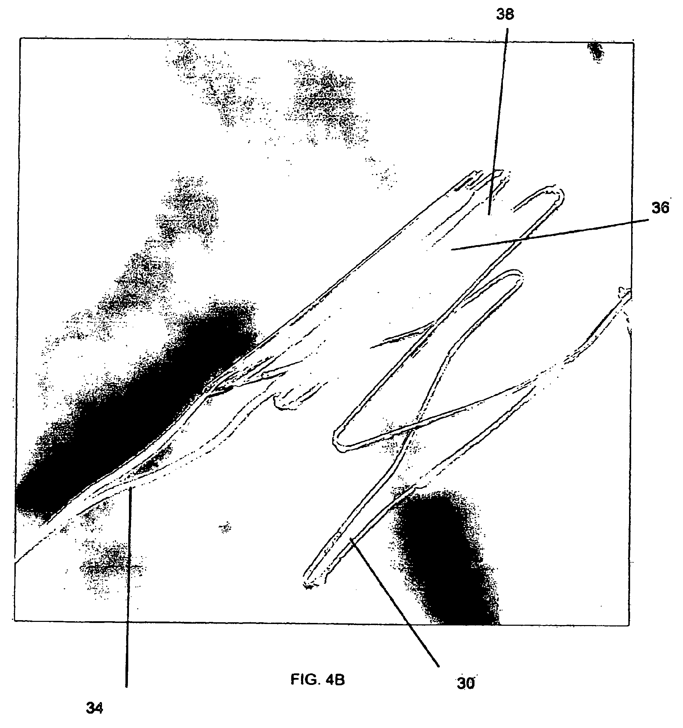

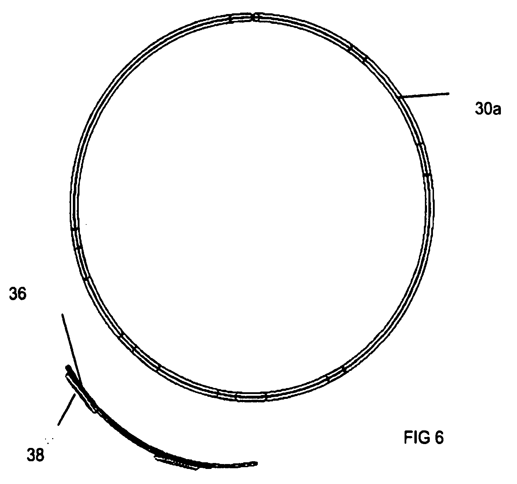

The flexible substrate material of the array 32a may include a plurality of tabs 39. The tabs are most easily seen in Fig. 5. During manufacture, tabs 39 are folded around struts of the anchor 30a and secured using an adhesive and/or a coating (e.g. polyurethane or silicone) applied to the substrate and tabs. Fig. 4A shows the assembly before the tabs 39have been folded over the struts of the anchor. Fig. 4B shows the assembly after the tabs have been folded. When the system is assembled, the electrodes 38 are positioned such that their conductive surfaces face away from the anchor 30a and will contact the inner wall of the target vessel when the anchor 30a is expanded, See Fig. 6.

The second array system is deployable from a sheath as discussed above. The delivery system used to deploy the system from the sheath may include a mechanism for maintaining engagement with the anchor during mapping, so that the anchor and array may be resheathed and repositioned during the mapping procedure.

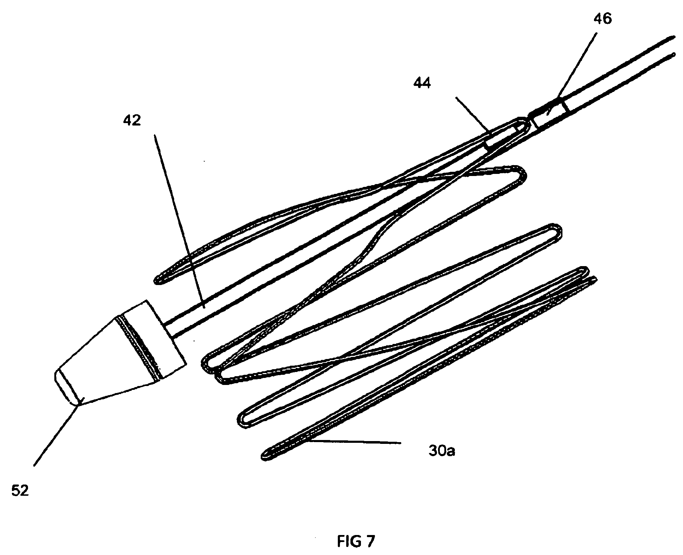

Referring to Fig. 7, in which the substrate is not shown for purposes of clarity, anchor 30a includes a u-shaped spline 40 that extends further proximal than the remainder of the anchor 30a. A shaft 42 for carrying the array/anchor assembly for deployment includes a distal stop 44 and a member 46. Anchor 30a is coupled to the delivery system by positioning a portion of spline (such as the U-bend at the proximal end) between the stop 44 and member 46, and by capturing the spline in this position using an inner sheath 48 as shown in Fig. 8. The anchor 30a is compressed within an outer sheath 50, which is slidably positioned over the inner sheath 48 as shown in Fig. 9. An atraumatic tip 52 may be positioned at the distal end of the shaft 42 as shown in Fig. 7. The tip 52 may include a hole or groove for receiving the distal end of the outer sheath 50 on its proximally-facing end to provide a smooth transition between the distal end of the outer sheath 50 and the tip 52.

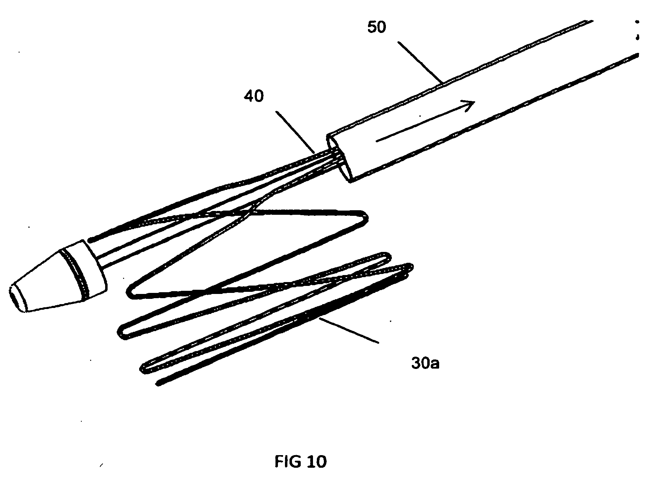

Prior to deployment of the system of the second embodiment, the components are arranged as shown in Fig. 9 and the system is percutaneously introduced and advanced to the target vessel and positioned at the predicted target location. Outer sheath 50 is withdrawn as indicated by the arrow in Fig. 10, to release the anchor 30a from the outer sheath 50, causing the anchor to expand and to position the electrodes in contact with the walls of the blood vessel. The inner sheath 48 remains positioned as in Fig. 8 so as to keep the connection between the shaft 42 and the anchor 30a.

The electrodes are activated and the response is measured. If the user wishes to test a different electrode position, the shaft 42 is rotated to change the rotational position of the electrodes within the vessel. The electrodes are activated and the response is measured in the second array position, and the process is repeated until an optimal electrode site is determined. Once the electrodes have been positioned at the optimal site, the inner sheath 48 is withdrawn, allowing the shaft 42 to be detached from the spline 40. The shaft 42 and sheaths are removed from the body, leaving the anchor and substrate in place.

### Third Embodiment

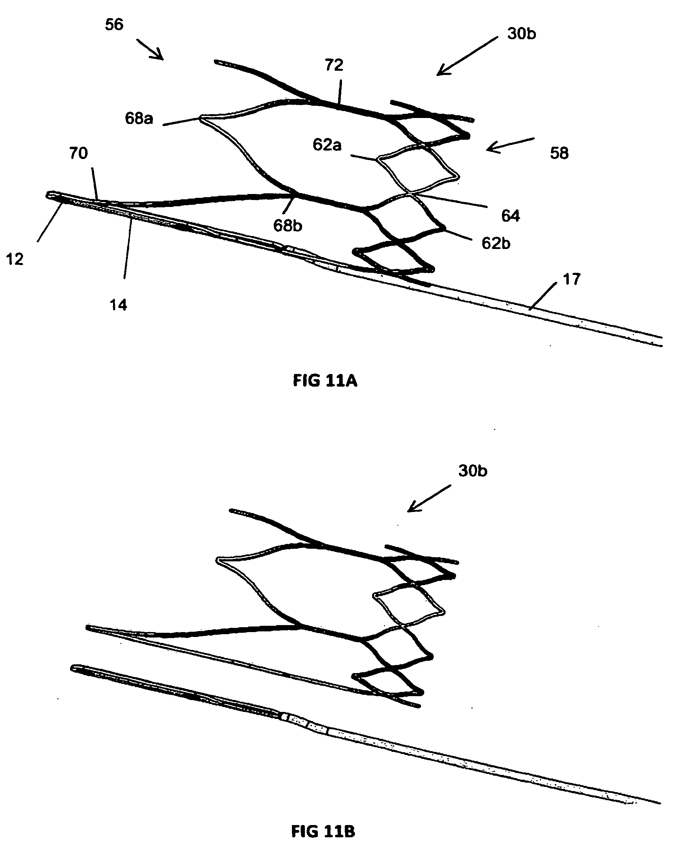

Figs. 11A through 12B illustrate an alternative anchor 30b in which temporary and chronic anchoring capabilities are integrated into a single structure. The anchor includes at least a first portion configured to retain the electrodes in position against the vessel wall for mapping, and a second portion that will chronically retain the implant at the chosen position within the vessel once the optimal array position has been selected. In a preferred embodiment, the anchor is an expandable stent-like sleeve, and the first anchor portion is positioned distally of the second anchor portion. The first and second portions are configured such that the radial expansion forces of the second portion are greater than the radial expansion forces of the first portion.

More particularly, anchor 30b includes a first portion 56 deployed during mapping to hold the electrodes in contact with the vessel wall using light enough radial expansion forces to allow for repositioning and/or re-sheathing of the electrodes as needed during a mapping procedure. A second portion 58 of the anchor is deployed after mapping has been completed and functions to firmly and chronically retain the electrodes at the chosen position. In a preferred anchor, the second portion 58 possesses radial forces sufficient to outwardly distend the vessel wall when it is deployed in the vessel, whereas the first portion 56 will not outwardly distend the vessel wall.

Anchor 30b is preferably formed of a length of tubing of resilient material such as nitinol that is laser cut to the desired pattern and shape set in its expanded shape. The patterns of first and second portions 56, 58 may take a variety of different forms. In the illustrated embodiment, second portion 58 includes a single circumferential ring in which the struts 60 form generally diamond-shaped openings. These openings are arranged with two corners 62a,b of the diamonds extending longitudinally in distal and proximal directions, and two corners 64 extending circumferentially. In a preferred embodiment, the angles between the struts 60 at the corners 62a, 62b are approximately 45 degrees or less.

The struts forming the first portion 56 of the illustrated embodiment includes struts 66a-c formed in an open, undulating pattern with distal peaks 68a, 70 and proximal peaks 68b. The struts 66a-c have a length that is longer than that of the struts 60 forming the diamond pattern of the second portion.

As shown, two of the distal peaks 70 extend further distally than the other distal peaks 68a. The struts 66b, 66c forming these peaks 70 support the electrodes 12 as will be discussed below.

Each of the proximal peaks 68b is connected by a longitudinal strut 72 to a longitudinally aligned distal corner 62a of the second portion 58. In an alternative embodiment, the strut 70 disposed between the longer distal peaks 70 may be omitted for reasons that are discussed below.

The design of the anchor results in a first section 56 that exerts smaller radial forces against the vessel wall than the second section 58 and that is more compressible in a radially inward direction than the second section. These different characteristics result from the fact that the first portion uses struts 66-c that are longer than those 60 of the second section - placing longer lever arms relative to the fold points in the first portion than in the second portion. Moreover, the first portion possess fewer such fold points than the second portion, making is less able to resist compression in response to radially inward forces imparted by the vessel wall.

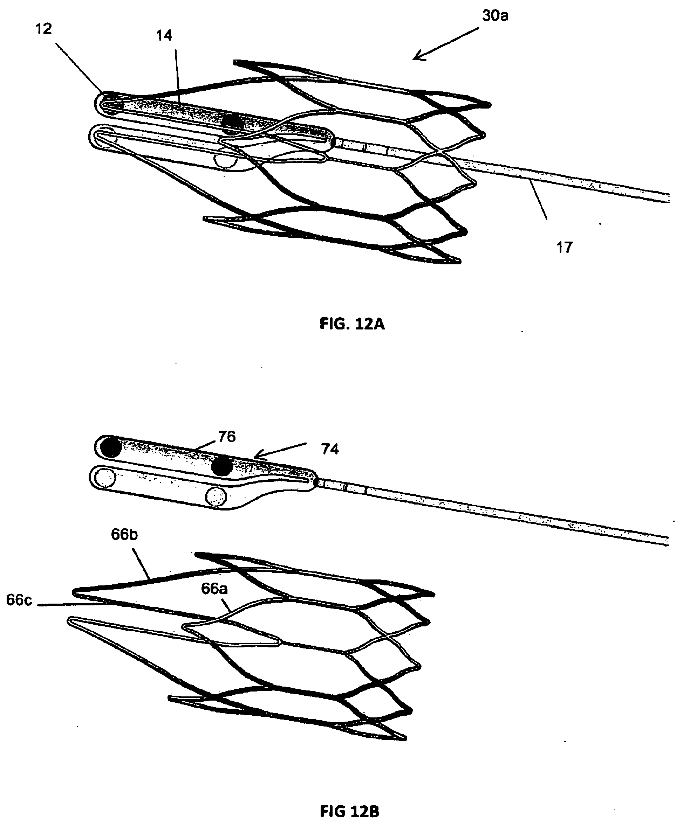

Electrodes of the type disclosed herein, or alternative forms of electrodes, are mounted to or formed on the anchor so as to contact the surrounding vessel wall when the anchor is expanded. Referring to Figs. 12A and 12B, in the embodiment as shown, electrodes 12 are formed on or molded into a flex circuit substrate 74 having a pair of parallel strips 76 on the distal end of lead 17. The lead 17 may be laterally offset from the longitudinal axis extending between and parallel to the strips 76 as shown, or it may extend along the longitudinal axis. The substrate preferably has properties similar to those disclosed above with respect to the first and second embodiments. Each strip includes at least one distal and one proximal electrode longitudinally aligned with one another, and larger numbers of electrodes may be used.

Referring to Fig. 12A, the substrates 74 are mounted to or molded onto struts 66b, 66c, and the electrodes 12 on the substrates are located distal to the cross-sectional plane defined by peaks 68b (in other words, distal to the struts 72). Struts 66b, 66c will preferably maintain the longitudinal alignment of each distal-proximal pair of electrodes. In the illustrated embodiment, each strut 66a angles outwardly from longitudinal strut 72, and from there extends parallel to the longitudinal axis of the anchor - causing the struts 66a to extend in parallel to one another.

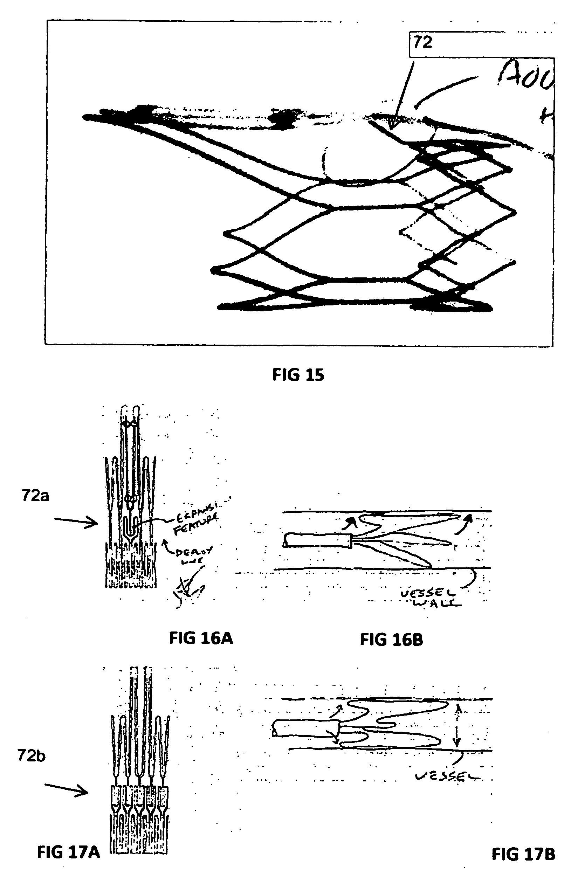

As shown in Fig. 15, the anchor and array of the third embodiment may be used in a fully intravascular system having an intravascular housing 78 containing a pulse generator, battery, and related circuitry and electronics.

In an alternative version of the device modified for acute use (such as to control heart rate or blood pressure through sympathetic and/or parasympathetic control of the autonomic nervous system during surgery), the second portion of the anchor may be eliminated, and the struts 72 mounted to the distal end of a catheter, with the first portion of the anchor extending from the struts as with the current embodiment.

In use, the integrated anchor is disposed within a delivery sheath prior to delivery into the vasculature. The sheath 50 may be similar to sheath 50 shown in Fig. 9. A tubular shaft 51 extends through the anchor and includes a member 53 in contact with the proximal portion of anchor for providing counter-traction during movement of the sheath relative to the anchor (for deployment and for resheathing when necessary). A small diameter tube 82 extends through the tubular shaft and includes a tip 52 similar to the tip described above. A lumen through the tube 82 and a corresponding lumen or opening in the tip 52 and allows the assembly (sheath, anchor, tubular shaft, small diameter tube) to be advanced through the vasculature over a guidewire. When assembled for delivery, the system may have an appearance similar to Fig. 9, with the sheath 50 and tip 52 forming an atraumatic outer assembly for smooth delivery through the vasculature.

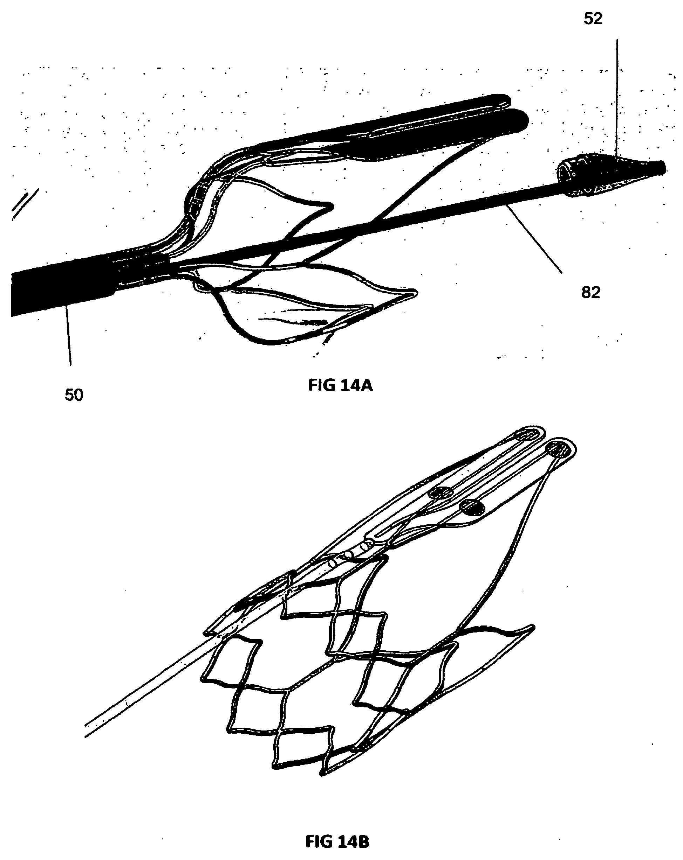

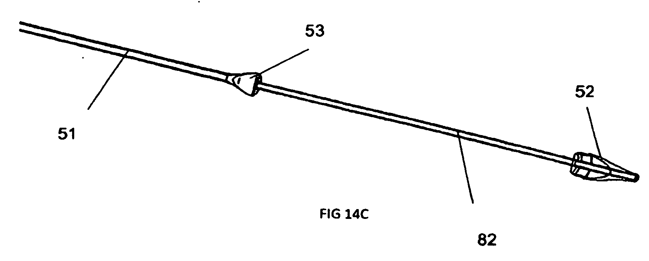

The sheath assembly is percutaneously introduced and advanced to a desired location with a target vessel (e.g. the superior vena cava for vagus nerve stimulation). The sheath is partially withdrawn while the member 53 holds the anchor in its longitudinal position within the vessel. The sheath is withdrawn until the first portion of the anchor is released from the sheath as shown in Fig. 14A, placing the electrodes into contact with the vessel wall. Mapping is performed at the target location. The properties of the first portion allow it to be resheathed by advancing the sheath while providing counter-traction using the lead 17 and the member 53/tubular shaft 51, and then repositioned and redeployed so that mapping may be carried out at additional sites if necessary. Once the optimal stimulation site is determined, the sheath is fully withdrawn to release the second portion of the anchor, thus firmly anchoring the electrodes at the chosen location within the blood vessel. The deployed second portion will preferably impart forces against the vessel wall that will cause the adjacent vessel wall to expand. Note that because the electrodes are positioned distal to the second portion of the anchor, this expansion of the vessel surround the second portion will not displace the electrodes from their target position

It is highly desirable during mapping to ensure that the electrodes fully contact the vessel walls. In a variation of the third embodiment, the strut 70 disposed between the longer distal peaks 70 is eliminated so that the proximally extending peak formed by struts 66c, 66c is not restrained by such a strut 72, which might otherwise limit the ability of the portions of the struts 66c near those peaks 70 (and the corresponding electrodes) to contact the vessel wall. Other embodiments shown in Figs. 15 through 17B use alternate configurations to ensure contact between the electrodes and the vessel wall during mapping and full deployment.

In particular, the Fig. 15 embodiment differs from the Fig. 11A embodiment in that strut 72 is shape set to extend away from the longitudinal axis of the anchor, so as to outwardly bias the proximal portions of substrate (and thus the electrodes). In the Fig. 16A, 16B embodiment, strut 72 is replaced by an expansion feature 72a biased such that when it is deployed from the sheath, it unfolds to advance the proximal end of the substrate towards the vessel wall. In the Figure 17A/17B embodiment, an expansion section is incorporated by adding the expansion feature of Fig. 16A to each of the struts 72, allowing each peak of the mapping portion of the anchor to more fully expand into contact with the surround vessel walls.

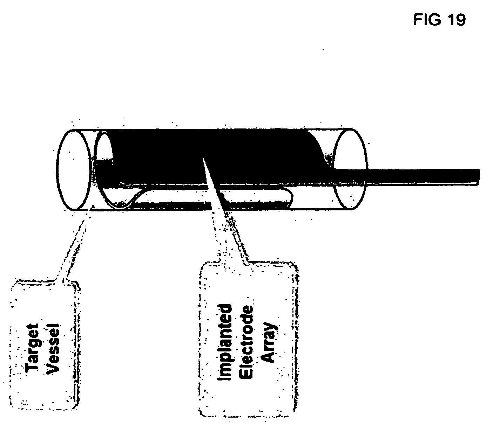

Fig. 18 shows an alternative substrate 14 and electrode 12 configuration that may be used for transvascular stimulation. Fig. 19 schematically shows the Fig. 18 embodiment in a curled position within a vessel. In this embodiment, the substrate may be deployed within the vessel in a rolled or spiral configuration. The substrate may be anchored in the vessel using an expandable anchor similar to those described herein, or expandable resilient baffles or expansion elements may be mounted to or molded into the substrate material.

The disclosed electrodes may be utilized for transvenous electrical stimulation from within the superior vena cava (SVC) or internal jugular vein to the vagus nerve to achieve reduction in blood pressure and heart rate, such as for treatment of congestive heart failure or other conditions.

### Examples

Animal studies were conducted in an effort to characterize parameters for electrical stimulation of the vagus nerve and non-target tissues. Results are presented in the graphs that follow this section. In animal models, multipolar electrode catheters were positioned at standard fluoroscopic sites within the right internal jugular vein, right brachiocephalic vein, right costocervicalis vein, and the SVC and used to stimulate the adjacent right vagus nerve and its cardiac branches through the vein wall.

Transvenous vagal neurostimulation with appropriate parameters and orientation reliably and reproducibly achieved Significant Cardiovascular Effect (defined as concurrent decrease in mean arterial pressure (MAP) > 10% and increase in R-R interval (duration of cardiac cycle) > 20%) at intravascular sites corresponding to the level of each cervical vertebrae C1-C7 and thoracic vertebrae T1-T3.

Threshold testing was conducted at each anatomic level from C1-T3 and systematic stimulus-response testing was performed for each of 3 key variables: intensity (current), frequency, and duration (pulse width).

Appropriate parameter selection enabled sustained significant cardiovascular effect up to 12 minutes (longest tested duration) without aberrant oscillation of heart rate and blood pressure; hemodynamic parameters typically returned to baseline within 30 seconds after stimulation without rebound tachycardia.

In no case did an animal become permanently refractory to vagal stimulation after significant cardiovascular effect was achieved, although vagal overdrive with supramaximal stimulation parameters transiently (<120 seconds) increased the stimulation threshold for significant cardiovascular effect in some cases.

Muscarinic blockade by administration of atropine abolished the cardiovascular effects of vagal nerve stimulation, indicating that these effects are mediated by efferent vagal parasympathetic fibers.

Collateral stimulation of non-target tissue predictably varied with stimulation location based on regional anatomy. Appropriate selection of stimulation parameters enabled achievement of significant cardiovascular effect without evidence of adverse effects (stimulation of other regional nerves or muscles) at all levels with the exception of C1 and C3, where stimulation invariably caused significant vibrations in the cervical musculature.

Stimulus response testing was conducted in canine models. Significant cardiovascular effects (reduction in MAP >10% increase in R-R interval > 20%) were achieved with transvenous vagal nerve stimulation at each anatomic level from C1-T3 at threshold currents ranging from 2.8-8.9 mA. Stimulation sites with threshold current requirements ≤ 6 mA were identified in each of the following vessels: right internal jugular vein, right brachiocephalic vein, right costocervicalis vein, and SVC. The lowest thresholds identified were at C3 (3.1 mA), C4 (3.4 mA), T3 (3.7 mA), and in the costocervicalis vein at the level of T2 (2.8 mA).

Increasing current above threshold produced an amplitude-dependent increase in cardiovascular effect. Strength-response curves are presented in Graphs 2 and 3. In the most effective frequency range (20-40 Hz), stimulation with supramaximal intensity reliably produced asystole that persisted for the duration of stimulation and spontaneously resolved upon its cessation. For every set of stimulation parameters that produced asystole, reduction in current intensity produced bradycardia instead.

Amplitude in the present study is primarily represented in terms of current (mA) rather than voltage (V) in order to facilitate direct comparison of the extracellular electric field generated by different multipolar electrode types with different average impedances at the electrode-tissue interface. The average impedances for the first electrode catheter (used here for transvenous vagal stimulation from the internal jugular vein) and the second electrode catheter (used for transvenous vagal stimulation from the SVC) were 730 Ω and 1150 Ω, respectively. For considerations of power consumption, it should be noted that significant cardiovascular effect was achieved at every level from C1-T3 at output voltages ranging from 2.5V - 8.8V (mean 4.8V). For reference, currently-available voltage-regulated pulse generators for neurostimulation are programmable to deliver voltage up to 10.5V.

Frequency-response testing at constant suprathreshold current and duration demonstrated progressive slowing of the cardiac cycle with increasing stimulation frequency up to 40 Hz. Response progressively deteriorated as frequency was further increased above 40 Hz (up to 100 Hz), presumably due to neural fatigue. Similarly, increasing frequency causes a progressive drop in MAP up to 20-40 Hz; further increasing frequency beyond this optimal range results in suboptimal MAP response. Frequency-response curves for transvenous vagal nerve stimulation are presented in Graphs 4 and 5.

Overall, frequencies between 10-40 Hz were found to be most reliable and effective for achieving significant cardiovascular effects using transvenous nerve stimulation. These findings are consistent with published reports of optimal stimulation frequencies for direct vagal nerve stimulation for heart rate reduction.

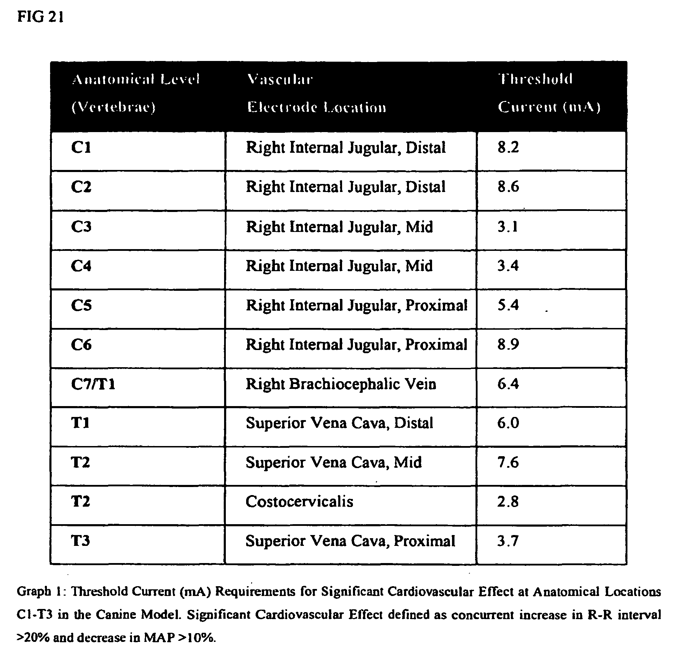

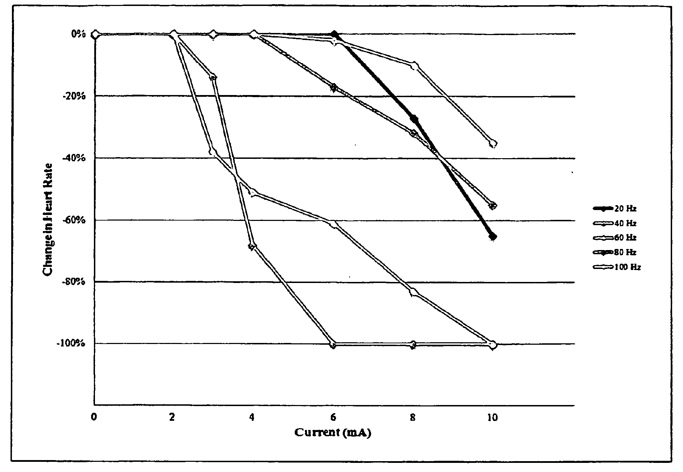

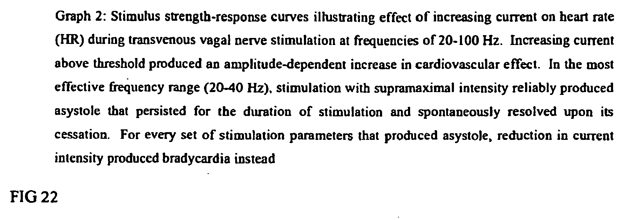

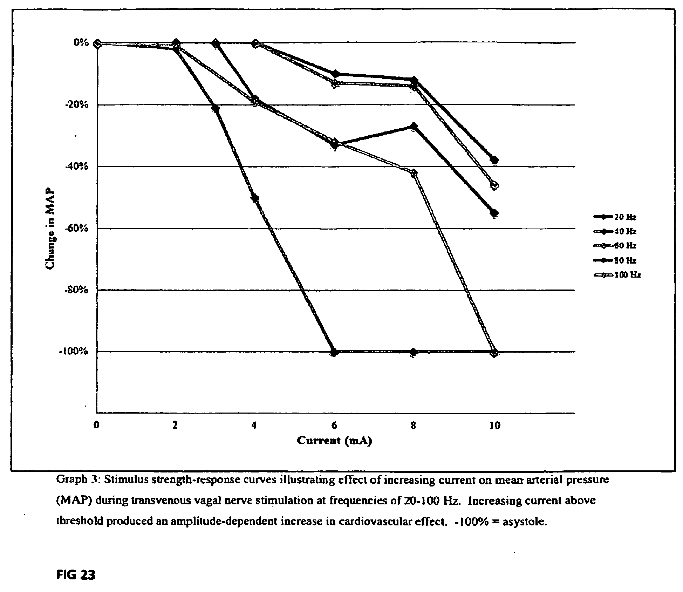

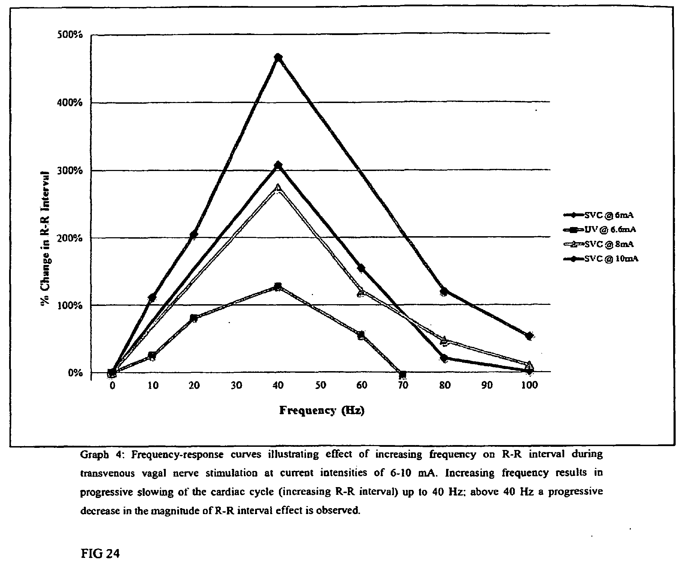

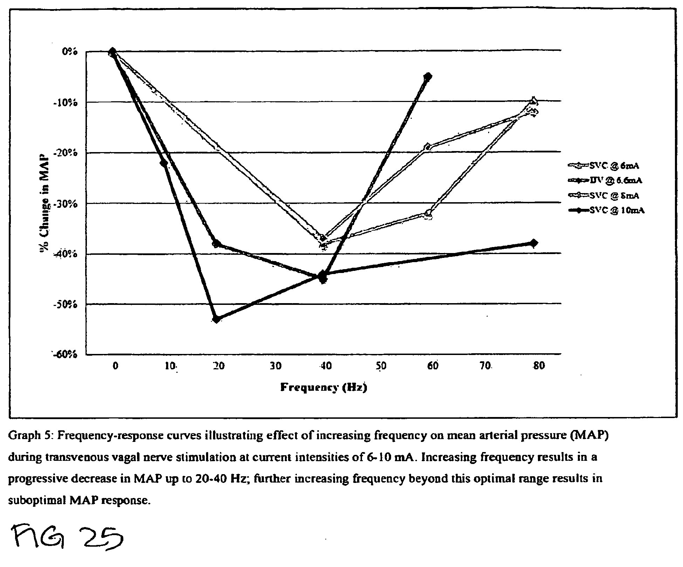

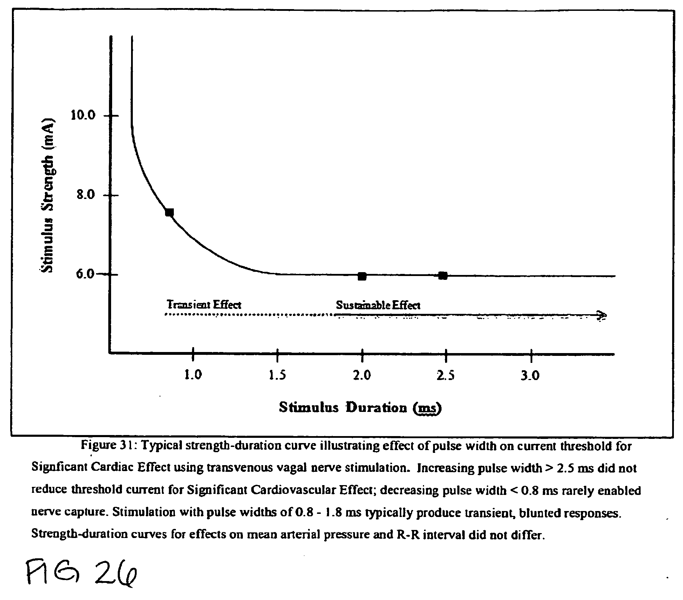

Strength-duration testing demonstrated reliable, reproducible cardiovascular effects of transvenous vagal nerve stimulation for pulse widths ≥ 2 ms (Graph 6). Increasing pulse width > 2.5 ms did not reduce threshold current for significant cardiovascular effect. Strength-duration curves for effects on MAP and R-R interval did not differ. Stimulation with pulse widths shorter than 1.8 ms routinely produced only transient, blunted cardiovascular responses. Capture was rarely observed for pulse widths < 0.5 ms.
Exemplary data is included in the graphs appearing at Figs. 21 - 24.

Additionally, the present disclosure contemplates a method of positioning an intravascular electrode array positioned on an expandable anchor, which does not form part of the present invention, and which comprises:
(a) providing a tubular sheath and further providing an anchor having a first portion and a second portion and an electrode array on the first portion, the anchor and electrode array in a compressed position within the sheath;
(b) advancing the sheath within a blood vessel and positioning the sheath at a location;
(c) at the location, withdrawing the sheath from a first portion of the anchor, causing the second portion to remain within the sheath and causing the first portion to expand and move the electrode array into contact with the walls of the blood vessel without distending the walls of the blood vessel;
(d) performing a mapping procedure using the electrodes at the first location;
(e) after performing the mapping procedure, recapturing the first portion into the sheath by advancing the sheath or withdrawing the anchor;
(f) moving the sheath to a second location;
(g) at the second location, withdrawing the sheath from the first portion of the anchor, causing the second portion to remain within the sheath and causing the first portion to expand and move the electrode array into contact with the walls of the blood vessel without distending the walls of the blood vessel;
(h) performing a mapping procedure using the electrodes at the second location;
(i) after step (h), withdrawing the sheath from the second portion of the anchor, causing the second portion to expand into contact with the walls of the blood vessel to chronically retain the electrodes at the second location and to retain the anchor in the vessel.

The scope of the invention is defined by the following claims.

## Claims

1. An intravascular mapping and anchoring system for chronic implantation within a blood vessel, comprising:
a sheath (50);
an expandable anchor (30b) comprising a first portion (56) having first radial expansion forces and a second portion (58) having second radial expansion forces greater than the first radial expansion forces, each portion having a compressed position and an expanded position;
an array of electrodes (12) on the first portion of the anchor;
wherein the anchor has a mapping position in which the second portion is in the compressed position and the first portion is in the expanded position to bias the electrodes against a blood vessel wall; and
wherein the anchor has an anchoring position in which the first portion is in the expanded position and the second portion is in the expanded position to engage the blood vessel wall for chronic retention of the anchor within the blood vessel; and
wherein the anchor has a delivery position in which the first and second portions are in their compressed positions within the sheath, the sheath being slidably retractable relative to the anchor to release the first portion from the compressed position and to place the anchor in the mapping position, and wherein when the anchor is in the mapping position, the sheath is further retractable to release the second portion from the compressed position to place the anchor in the anchoring position.

2. The system of claim 1, wherein when the anchor is in the mapping position each electrode is at an electrode location on the vessel wall, and wherein the second portion is expandable to move the anchor to the anchoring position without displacing the electrodes from their electrode locations.

3. The system of claim 1, wherein the first portion is comprised of a plurality of first struts (66a-c) and the second portion is comprised of a plurality of second struts (60), and wherein the first struts have a length that is longer than the length of the second struts.

4. The system of claim 1 further including a flexible substrate (74) carried by the first portion, wherein the electrodes are disposed on the flexible substrate.

5. The system of claim 4 wherein the substrate is formed of polyimide.

6. The system of claim 4 wherein the substrate is formed of silicone.

7. The system of claim 4, further including a coating on the substrate.

8. The system of claim 7 wherein the coating is formed of polyurethane.

9. The system of claim 1, wherein:
the anchor is advanceable within a blood vessel with the first and second portions in the compressed position;
the first portion is expandable to the expanded position to bias the electrodes in contact with the walls of the blood vessel at a first location within the blood vessel to permit a mapping procedure using the electrodes at the first location while the second portion of the anchor is maintained in the compressed position;
the anchor is repositionable within the blood vessel to place the electrodes at a second location different from the first location to permit a mapping procedure using the electrodes at the second location while the second portion is maintained in the compressed position; and
wherein the second portion is moveable to the expanded position to chronically retain the electrodes at the second location.

10. The system of claim 9, wherein the first portion of the anchor is moveable to the compressed position at the first location for repositioning of the anchor within the blood vessel, and moveable to expanded position at the second location.

11. The system of claim 1, wherein the radial expansion forces of the second portion of the anchor are sufficient to distend walls of a surrounding blood vessel in a radially outward direction, and the radial expansion forces of the first portion of the anchor are insufficient to distend surrounding blood vessel walls in a radially outward direction.

## Patentansprüche

1. Intravaskuläres Abbildungs- und Verankerungssystem zur chronischen Implantation in einem Blutgefäß, das Folgendes umfasst:
einen Mantel (50);
einen ausdehnbaren Anker (30b), der einen ersten Abschnitt (56) mit ersten radialen Ausdehnungskräften und einen zweiten Abschnitt (58) mit zweiten radialen Ausdehnungskräften umfasst, die größer sind als die ersten radialen Ausdehnungskräfte, wobei jeder Abschnitt eine zusammengedrückte Position und eine ausgedehnte Position hat;
eine Gruppe von Elektroden (12) auf dem ersten Abschnitt des Ankers;
wobei der Anker eine Abbildungsposition hat, in der der zweite Abschnitt in der zusammengedrückten Position ist und der erste Abschnitt in der ausgedehnten Position ist, um die Elektroden gegen eine Blutgefäßwand vorzuspannen; und
wobei der Anker eine Verankerungsposition hat, in der der erste Abschnitt in der ausgedehnten Position ist und der zweite Abschnitt in der ausgedehnten Position ist, um die Blutgefäßwand zum chronischen Festhalten des Ankers in dem Blutgefäß in Eingriff zu bringen; und
wobei der Anker eine Zuführungsposition hat, in der der erste und der zweite Abschnitt in ihren zusammengedrückten Position in dem Mantel sind, wobei der Mantel relativ zum Anker gleitend zurückgezogen werden kann, um den ersten Abschnitt aus der zusammengedrückten Position zu lösen und den Anker in der Abbildungsposition zu platzieren, und wobei, wenn der Anker in der Abbildungsposition ist, der Mantel weiter zurückgezogen werden kann, um den zweiten Abschnitt von der zusammengedrückten Position zu lösen, um den Anker in der Verankerungsposition zu platzieren.

2. System nach Anspruch 1, wobei sich jede Elektrode, wenn der Anker in der Abbildungsposition ist, an einer Elektrodenstelle an der Gefäßwand befindet, und wobei der zweite Abschnitt ausdehnbar ist, um den Anker in die Verankerungsposition zu bewegen, ohne die Elektroden von ihren Elektrodenstellen zu verschieben.

3. System nach Anspruch 1, wobei der erste Abschnitt aus mehreren ersten Streben (66a-c) besteht und der zweite Abschnitt aus mehreren zweiten Streben (60) besteht und wobei die ersten Streben länger sind als die zweiten Streben.

4. System nach Anspruch 1, das ferner ein vom ersten Abschnitt getragenes flexibles Substrat (74) aufweist, wobei die Elektroden auf dem flexiblen Substrat angeordnet sind.

5. System nach Anspruch 4, wobei das Substrat aus Polyimid gebildet ist.

6. System nach Anspruch 4, wobei das Substrat aus Silikon gebildet ist.

7. System nach Anspruch 4, das ferner eine Beschichtung auf dem Substrat aufweist.

8. System nach Anspruch 7, wobei die Beschichtung aus Polyurethan gebildet ist.

9. System nach Anspruch 1, wobei:
der Anker in einem Blutgefäß vorgeschoben werden kann, mit dem ersten und dem zweiten Abschnitt in der zusammengedrückten Position;
der erste Abschnitt in die ausgedehnte Position ausgedehnt werden kann, um die Elektroden in Kontakt mit den Wänden des Blutgefäßes an einer ersten Stelle in dem Blutgefäß vorzuspannen, um ein Abbildungsverfahren mit den Elektroden an der ersten Stelle zuzulassen, während der zweite Abschnitt des Ankers in der zusammengedrückten Position gehalten wird;
der Anker in dem Blutgefäß umpositioniert werden kann, um die Elektroden an einer zweiten Stelle zu platzieren, die sich von der ersten Stelle unterscheidet, um ein Abbildungsverfahren mit den Elektroden an der zweiten Stelle zuzulassen, während der zweite Abschnitt in der zusammengedrückten Position gehalten wird; und
der zweite Abschnitt in die ausgedehnte Position bewegt werden kann, um die Elektroden chronisch an der zweiten Stelle festzuhalten.

10. System nach Anspruch 9, wobei der erste Abschnitt des Ankers in die zusammengedrückte Position an der ersten Stelle bewegt werden kann, um den Anker in dem Blutgefäß umzupositionieren, und in die ausgedehnte Position an der zweiten Stelle bewegt werden kann.

11. System nach Anspruch 1, wobei die radialen Ausdehnungskräfte des zweiten Abschnitts des Ankers ausreichen, um Wände eines umgebenden Blutgefäßes in einer radial auswärtigen Richtung auszudehnen, und die radialen Ausdehnungskräfte des ersten Abschnitts des Ankers nicht ausreichen, um umgebende Blutgefäßwände in einer radial auswärtigen Richtung auszudehnen.

## Revendications

1. Système de cartographie et d'ancrage intravasculaire pour implantation chronique dans un vaisseau sanguin, comprenant :
un fourreau (50) ;
une ancre expansible (30b) comprenant une première partie (56) ayant des premières forces d'expansion radiale et une deuxième partie (58) ayant des deuxièmes forces d'expansion radiale plus grandes que les premières forces d'expansion radiale, chaque partie ayant une position comprimée et une position d'expansion ;
une série d'électrodes (12) sur la première partie de l'ancre ;
où l'ancre a une position de cartographie dans laquelle la deuxième partie est dans la position comprimée et la première partie est dans la position d'expansion pour incliner les électrodes contre une paroi de vaisseau sanguin ; et
où l'ancre a une position d'ancrage dans laquelle la première partie est dans la position d'expansion et la deuxième partie est dans la position d'expansion afin d'engager la paroi de vaisseau sanguin pour la rétention chronique de l'ancre dans le vaisseau sanguin; et
où l'ancre a une position de largage dans laquelle la première et la deuxième partie sont dans leur position comprimée à l'intérieur du fourreau, le fourreau étant rétractable en coulissant par rapport à l'ancre pour libérer la première partie de la position comprimée et pour placer l'ancre dans la position de cartographie, et où lorsque l'ancre est dans la position de cartographie, le fourreau est encore rétractable pour libérer la deuxième partie de la position comprimée afin de placer l'ancre dans la position d'ancrage.

2. Système selon la revendication 1, dans lequel lorsque l'ancre est dans la position de cartographie chaque électrode est à un emplacement d'électrode sur la paroi du vaisseau, et où la deuxième partie est expansible pour déplacer l'ancre à la position d'ancrage sans déplacer les électrodes de leurs emplacements d'électrodes.

3. Système selon la revendication 1, dans lequel la première partie est constituée d'une pluralité de premiers étrésillons (66a-c) et la deuxième partie est constituée d'une pluralité de deuxièmes étrésillons (60), et où les premiers étrésillons ont une longueur qui est plus longue que la longueur des deuxièmes étrésillons.

4. Système selon la revendication 1, comprenant en outre un substrat flexible (74) porté par la première partie, où les électrodes sont disposées sur le substrat flexible.

5. Système selon la revendication 4, dans lequel le substrat est formé en polyimide.

6. Système selon la revendication 4, dans lequel le substrat est formé en silicone.

7. Système selon la revendication 4, comprenant en outre un revêtement sur le substrat.

8. Système selon la revendication 7, dans lequel le revêtement est formé en polyuréthane.

9. Système selon la revendication 1, dans lequel :
l'ancre peut être avancée dans un vaisseau sanguin avec la première et la deuxième partie dans la position comprimée ;
la première partie est expansible à la position d'expansion pour incliner les électrodes en contact avec les parois du vaisseau sanguin à un premier emplacement dans le vaisseau sanguin afin de permettre une procédure topographique en utilisant les électrodes au premier emplacement pendant que la deuxième partie de l'ancre est maintenue dans la position comprimée ;
l'ancre peut être repositionnée dans le vaisseau sanguin pour placer les électrodes à un deuxième emplacement différent du premier emplacement afin de permettre une procédure topographique en utilisant les électrodes au deuxième emplacement pendant que la deuxième partie est maintenue dans la position comprimée ; et
dans lequel la deuxième partie est déplaçable à la position d'expansion afin de retenir chroniquement les électrodes au deuxième emplacement.

10. Système selon la revendication 9, dans lequel la première partie de l'ancre est déplaçable à la position comprimée au premier emplacement afin de repositionner l'ancre dans le vaisseau sanguin, et déplaçable à la position d'expansion au deuxième emplacement.

11. Système selon la revendication 1, dans lequel les forces d'expansion radiale de la deuxième partie de l'ancre sont suffisantes pour étirer les parois d'un vaisseau sanguin environnant dans une direction radialement vers l'extérieur, et les forces d'expansion radiale de la première partie de l'ancre sont insuffisantes pour étirer les parois d'un vaisseau sanguin environnant dans une direction radialement vers l'extérieur.
